(19) Europäisches Patentamt
European Patent Office
Office européen des brevets

(11) EP 3 059 300 B1

(12) **FASCICULE DE BREVET EUROPEEN**

(45) Date de publication et mention
de la délivrance du brevet:
**11.08.2021 Bulletin 2021/32**

(51) Int Cl.:
**C12M 1/00** *(2006.01)*          **C12M 3/06** *(2006.01)*
**C12M 1/12** *(2006.01)*          **C12M 1/26** *(2006.01)*

(21) Numéro de dépôt: **16156161.8**

(22) Date de dépôt: **17.02.2016**

(54) **DISPOSITIF DE MANIPULATION DE CELLULES BIOLOGIQUES AU MOYEN D'UN SUPPORT VIBRANT**

VORRICHTUNG ZUR MANIPULATION VON BIOLOGISCHEN ZELLEN MITHILFE EINER SCHWINGENDEN HALTERUNG

DEVICE FOR MANIPULATING BIOLOGICAL CELLS USING A VIBRATING HOLDER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.02.2015 FR 1551409**

(43) Date de publication de la demande:
**24.08.2016 Bulletin 2016/34**

(73) Titulaire: **Commissariat à l'Énergie Atomique et aux Énergies Alternatives 75015 Paris (FR)**

(72) Inventeurs:
• **CASSET, Fabrice**
  **38570 TENCIN (FR)**
• **MILLET, Arnaud**
  **38340 Voreppe (FR)**

(74) Mandataire: **Brevalex**
  **95, rue d'Amsterdam**
  **75378 Paris Cedex 8 (FR)**

(56) Documents cités:
| | |
|---|---|
| **EP-A1- 2 747 452** | **WO-A1-2009/018847** |
| **WO-A1-2014/037862** | **WO-A2-2007/053281** |
| **WO-A2-2012/027366** | **WO-A2-2012/140519** |
| **US-A1- 2008 245 745** | **US-A1- 2012 273 357** |
| **US-A1- 2012 276 622** | |

• **ZHONGLAN TANG ET AL: "Shear stress-dependent cell detachment from temperature-responsive cell culture surfaces in a microfluidic device", BIOMATERIALS., vol. 33, no. 30, 20 juillet 2012 (2012-07-20), pages 7405-7411, XP055263558, GB ISSN: 0142-9612, DOI: 10.1016/j.biomaterials.2012.06.077**
• **DEBAVELAERE-CALLENS ET AL: "On the use of ultrasounds to quantify the longitudinal threshold force to detach osteoblastic cells from a conditioned glass substrate", BIOMOLECULAR ENGINEERING, ELSEVIER, NEW YORK, NY, US, vol. 24, no. 5, 1 novembre 2007 (2007-11-01), pages 521-525, XP022356615, ISSN: 1389-0344, DOI: 10.1016/J.BIOENG.2007.08.016**

EP 3 059 300 B1

**Description**

**DOMAINE TECHNIQUE ET ÉTAT DE LA TECHNIQUE ANTÉRIEURE**

La présente invention se rapporte à un dispositif de manipulation de cellules biologiques au moyen d'un support vibrant et à un dispositif de tri de cellules biologiques. La possibilité de trier les cellules biologiques à partir d'un mélange hétérogène est un enjeu majeur en biomédecine.

Il existe deux techniques principalement utilisées :

- le tri par cytométrie en flux (FACS : Fluorescence Activated Cell Sorting en terminologie anglo-saxonne), cette technique repose sur le marquage fluorescent des cellules à l'aide de colorants spécifiques ou d'anticorps fluorescents dirigés contre des antigènes de surface. Les propriétés de diffusion de la lumière et de fluorescence permettent de sélectionner la ou les populations d'intérêts. Cette technique offre la possibilité d'une sélection avec une pureté élevée mais au prix d'un équipement couteux.
- l'isolement à partir de billes magnétiques (MACS : MagneticActivatedCellSortingen terminologie anglo-saxonne), cette technique repose sur l'utilisation d'anticorps dirigés contre des antigènes de surface couplés à des billes magnétiques permettant un débit cellulaire pouvant dépasser la cytométrie en flux au prix d'une pureté moindre.

Ces deux techniques sont très utilisées mais nécessitent l'utilisation de cellules en suspension, requièrent une mise au point concernant la concentration de l'anticorps, la densité de cellules, la taille des billes magnétiques et nécessite l'existence de marqueurs spécifiques exprimés à la surface de la cellule permettant le tri. Or ces marqueurs peuvent se révéler inexistant pour certains types de cellules, par exemple pour les cellules cancéreuses qui ne présentent pas toujours d'antigènes de surface spécifiques identifiés.

Par conséquent, ces techniques de tri ou d'isolement ne sont pas applicables à toutes les cellules.

Il existe d'autres techniques permettant un tri sans marquage préalable, par exemple les techniques utilisant la taille et la densité (CCE Counterflow Centrifugal Elutriation en terminologie anglo-saxonne) et celles utilisant le fractionnement par dielectrophorèse. En revanche, elles ne permettant pas d'atteindre une résolution suffisante. En outre, dans le cas particulier de la diélectrophorèse, la survie des cellules ne peut être assurée.

Un autre paramètre physique est utilisé pour trier les cellules, il s'agit de l'adhérence cellulaire par rapport à une surface, qui dépend du type cellulaire étudié, de la surface d'adhérence et de l'environnement biochimique de la cellule. Ce paramètre physique peut se révéler très discriminant pour le tri cellulaire sans nécessiter de marquage préalable.

Ce paramètre physique est utilisé dans des dispositifs de chromatographie cellulaire d'affinité comportant une chambre microfluidique, dans lesquels une surface est fonctionnalisée. Un fluide transportant des cellules s'écoule sur une surface fonctionnalisée. Les cellules adhèrent à la surface suivant leur affinité avec la surface.

La fonctionnalisation de la surface peut se faire de manière chimique par formation d'une couche de fonctionnalisation ou par nanostructuration. Le document Chang et al Biomimetic technique for adhesion-based collection and separation of cells in a microfluidic channel. Lab Chip 2005, 5, 64-73 décrit un tel dispositif. Le document Mandrusov et al. Membrane-based cell affinity chromatography to retrieve viable cells. Biotechnol.Prog. 1995, 11, 208-213 décrit également une méthode de séparation cellulaire utilisant l'affinité avec une surface.

Le document Sin et al. Enrichment using antibody-coated microfluidic chambers in shear flow: model mixtures of human lymphocytes. Biotechnology & Bioengineering, VOL.91, NO.7, September30, 2005 pages 816-826 décrit l'utilisation de circuits micro-fluidiques avec des surfaces fonctionnalisées par des anticorps. Le document Kwon et al. "Label-free, microfluidic separation and enrichment of human breast cancer cells by adhesion difference" Lab Chip 2007, 7, 1461-1468 décrit l'utilisation de circuits micro-fluidiques avec surfaces fonctionnalisées par des surfaces structurées.

Ces dispositifs permettent de collecter des cellules présentant une affinité avec la surface fonctionnalisée, mais la récupération de ces cellules peut poser problème. La récupération peut être obtenue en appliquant une contrainte de cisaillement au moyen d'un liquide de sorte à décrocher les cellules. Ces dispositifs ne permettent pas l'application d'une force de cisaillement uniforme, notamment sur les bords des chambres microfluidiques, empêchant ainsi un tri efficace. WO2012/140519 divulgue un dispositif comportant un support 3D permettant l'adhérence desdites cellules, un actionneur apte à mettre en vibration ladite surface à au moins une fréquence donnée provoquant le détachement d'au moins un type de cellules biologiques. WO2012/140519 ne décrit pas de membrane suspendue.

**EXPOSÉ DE L'INVENTION**

C'est par conséquent un but de la présente invention d'offrir un dispositif offrant une manipulation de cellules biologiques facilitée et pouvant être discriminante.

C'est un but supplémentaire de la présente invention d'offrir un dispositif de tri cellulaire offrant une efficacité améliorée, notamment dans le tri des cellules cancéreuses.

Le but énoncé ci-dessus est atteint par un dispositif mettant en œuvre un support et des moyens de mise en vibration du support suivant au moins une fréquence de sorte à appliquer une force inertielle aux cellules qui ont adhéré au support qui est apte à la décrocher. La force d'adhérence à une surface donnée permet de caractériser un type de cellules par rapport à un autre. On peut ainsi modifier l'adhérence au support des cellules en modifiant la fréquence de vibration et décrocher un type de cellule souhaité.

En d'autres termes, on réalise un dispositif présentant des propriétés d'adhérence différentes vis-à-vis de types cellulaires différents, ces différents types cellulaires sont sélectionnés en leur imprimant une force qui est suffisante pour détacher du support une population cellulaire donnée.

Par exemple, la mise en vibration du support est obtenue au moyen d'un actionneur piézoélectrique.

Par exemple, on fait circuler un fluide contenant des cellules d'intérêt sur un support, les cellules d'intérêt et d'autres adhèrent au support. En appliquant une ou des vibrations de fréquences données, on peut ensuite soit détacher uniquement les cellules d'intérêt soit uniquement les autres cellules. En ne conservant que les cellules d'intérêt sur le support, on peut accumuler les cellules d'intérêt sur le support.

De manière avantageuse, la ou les surfaces de support peut ou peuvent être fonctionnalisée(s) pour modifier les forces d'adhérence des cellules sur la ou les surfaces.

La présente invention a alors pour objet un dispositif de manipulation d'un ou de plusieurs types de cellules biologiques distinguables à partir de leurs propriétés d'adhérence, comportant :

- au moins un support comprenant une surface d'accueil permettant l'adhérence desdites cellules, ledit support étant formé par une membrane suspendue, chaque type de cellules biologiques présentant une force d'adhérence pour la surface d'accueil,
- au moins un actionneur apte à mettre en vibration ladite surface à au moins une fréquence propre de la membrane, et
- des moyens de commande dudit actionneur configurés au moins pour mettre en vibration la surface d'accueil à une fréquence propre de la membrane déterminée en fonction de la force d'adhérence d'un type de cellules biologiques provoquant le détachement dudit type de cellules biologiques.

Dans la présente demande, on entend par "type de cellules" soit une espèce cellulaire soit un état biologique particulier au sein d'une même espèce.

Grâce à ce dispositif, le détachement d'au moins un type de cellules biologiques permet notamment leur distinction

Dans le cas de cellules biologiques de plusieurs types, les moyens de commande peuvent commander l'actionneur de sorte qu'il puisse mettre en vibration ladite surface à plusieurs fréquences données, chacune des fréquences étant choisie de sorte à provoquer le détachement d'au moins un des types de cellules biologiques.

De manière préférée, la ou les fréquences est ou sont choisie(s) de sorte que les ondes générées correspondent à des déformations de la surface de l'ordre de la taille des cellules biologiques ou inférieures à la taille des cellules biologiques.

Un dispositif dans lequel les ondes générées correspondent à des déformations de la surface supérieures à la taille des cellules biologiques ne sort pas du cadre de la présente invention.

Dans un exemple de réalisation, le dispositif est apte à mettre en vibration la surface à plusieurs fréquences, lesdites différentes fréquences étant appliquées de préférence séquentiellement.

Tout ou partie de la surface peut être fonctionnalisée de sorte à modifier la force d'adhérence du ou des types de cellules biologiques par rapport à la surface non fonctionnalisée.

Dans un exemple de réalisation, le support est formé par une membrane suspendue. La plus grande dimension de la membrane peut être comprise entre quelques $\mu$m et quelques centaines de $\mu$m, de préférence entre 5 $\mu$m et 20000 $\mu$m.

Dans un autre exemple de réalisation, le support est formé par une plaque, par exemple une plaque en verre, dont les côtés mesurent par exemple entre quelques centaines de $\mu$m à quelques cm

Avantageusement, les moyens de commande commandent l'actionneur de sorte que le support vibre dans un mode de Lamb.

Plusieurs actionneurs peuvent être répartis sur ou sous la surface du support. Les moyens de commande peuvent commander les actionneurs de sorte qu'ils mettent chacun en vibration la surface du support selon un mode différent.

L'actionneur ou les actionneurs peuvent être choisis parmi des actionneurs piézoélectriques, ferroélectriques, électrostatiques, magnétiques ou thermiques. On peut prévoir d'utiliser différents types d'actionneur sur le même support.

Dans un exemple de réalisation, l'actionneur est réalisé sur une face du support opposée à la surface destinée à entrer en contact avec les cellules biologiques.

De manière très avantageuse, le dispositif de manipulation est un dispositif MEMS et/ou NEMS.

La présente invention a également pour objet un dispositif de tri de cellules biologiques comportant au moins un dispositif de manipulation de cellules biologiques selon l'invention.

La présente invention a également pour objet un dispositif microfluidique comportant au moins un dispositif de manipulation de cellules biologiques selon l'invention ou un dispositif de tri selon l'invention, comportant au moins une entrée d'alimentation en une solution comportant au moins un type de cellules biologiques et au moins une sortie d'évacuation, le support étant disposé entre l'entrée d'alimentation et la sortie d'évacuation.

Le dispositif microfluidique peut-être utiliser pour faire du tri de cellules ou par exemple pour du nettoyage de surface de support

La présente invention a également pour objet un procédé de tri de différents types de cellules biologiques contenus dans une solution mettant en œuvre le dispositif de tri selon l'invention, comportant les étapes:

- mise en contact de la solution contenant les différents types de cellules biologiques avec la surface du support,
- adhérence des cellules de différents types sur la surface du support,
- mise en vibration de la surface à au moins une fréquence donnée de sorte à détacher au moins l'un des types de cellules,
- évacuation des cellules détachées.

## BRÈVE DESCRIPTION DES DESSINS

La présente invention sera mieux comprise sur la base de la description qui va suivre et des dessins en annexe sur lesquels:

- la figure 1 est une représentation schématique d'un dispositif de tri cellulaire mettant en œuvre l'invention,
- la figure 2 est une vue en coupe d'un exemple de support pouvant être mis en œuvre dans le dispositif de la figure 1,
- la figure 3 est une vue de trois quarts d'un autre exemple de support muni de plusieurs actionneurs,
- la figure 4 est une représentation du support de la figure 3 dans différents modes de vibration,
- la figure 5 est une vue de trois quarts d'un autre exemple de support muni de plusieurs actionneurs pour une mise en vibrations dans différents modes,
- les figures 6A et 6B sont des vues du support de la figure 5 dans différents modes de vibration,
- les figures 7A à 7S sont des représentations schématiques des éléments obtenus au cours des différentes étapes d'un exemple de procédé de réalisation d'un dispositif de tri,
- la figure 8 représente des modes de vibration possible d'une membrane circulaire.

## EXPOSÉ DÉTAILLÉ DE MODES DE RÉALISATION PARTICULIERS

La description qui va suivre porte principalement sur l'application de l'invention au tri de cellules vivantes mais la présente invention peut avoir d'autres applications qui seront décrites ci-après.

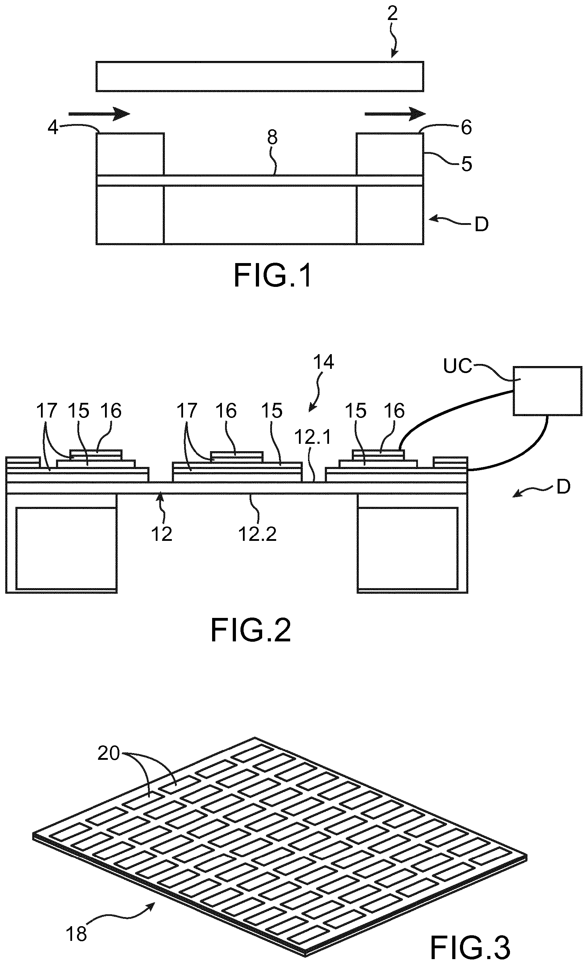

Sur la figure 1, on peut voir une représentation schématique d'un exemple d'un dispositif de tri cellulaire mettant en œuvre la présente invention.

Dans l'exemple représenté, le dispositif de tri cellulaire comporte un boîtier 2 muni d'une entrée d'alimentation fluidique 4 réalisée dans une paroi latérale 5 du boîtier et d'une sortie d'évacuation fluide 6 réalisée dans la paroi latérale 5 du boîtier et un fond 8. L'entrée d'alimentation 4 et la sortie d'évacuation 6 sont disposées par rapport au fond 8 de sorte que le flux de fluide s'écoule sur le fond de l'entrée 4 vers la sortie 6.

On peut envisager de disposer le dispositif dans une enceinte avec température contrôlée.

Le fond 8 est formé par un dispositif D offrant des propriétés d'adhérence variables pour au moins un type de cellule.

Le dispositif D comporte un support, dans l'exemple représenté, formé par une membrane suspendue 12 à la paroi latérale du boîtier. Le dispositif comporte également au moins un actionneur apte à mettre en vibration la membrane suivant une fréquence donnée. Dans l'exemple représenté, le dispositif comporte deux actionneurs 14, 16 mettant en œuvre un matériau ferroélectrique, par exemple du PZT, l'un des actionneurs sert au déplacement de la membrane vers le haut et l'autre actionneur sert au déplacement de la membrane vers le bas. Dans le cas où un matériau piézoélectrique est mis en œuvre, un seul actionneur est suffisant pour assurer les déplacements vers le haut et vers le bas, puisqu'un matériau piézoélectrique se dilate et se contracte suivant le signe de la tension appliquée.

Le dispositif D est représenté de manière isolée sur la figure 2, celui-ci étant retourné par rapport à la représentation de la figure 1.

La membrane 12 a une forme circulaire, l'actionneur 14 est un actionneur ferroélectrique et est disposé sensiblement au centre de la membrane 12 sur une face 12.1 opposée à celle 12.2 située dans le boîtier et destinée à entrer en contact avec le fluide. L'actionneur 16 est également un actionneur ferroélectrique de forme annulaire et bordant le bord extérieur de la surface extérieure 12.1 de la membrane 12.

La surface intérieure 12.2 est telle qu'elle présente des propriétés d'adhérence par rapport à un ou plusieurs types de cellules. La surface 12.2 est par exemple en verre, en silicium ou tout matériau semi-conducteur.

En variante, on pourrait envisager que ce soit le côté du dispositif portant le ou les actionneurs qui présente des propriétés d'adhérence par rapport à un ou plusieurs types de cellules; des moyens pour isoler électriquement les actionneurs du fluide contenant les cellules seraient alors prévus, par exemple une couche isolante électrique.

Les actionneurs 14, 16 comportent un matériau ferroélectrique 15 et deux électrodes 17 de part et d'autres du matériau 15 permettant d'appliquer à ce matériau 15 un champ électrique sur commande, qui provoque une déformation dans le plan du matériau et donc une déformation hors plan de la membrane dont il est solidaire, par effet bilame. En appliquant une tension alternative aux électrodes 17, la membrane vibre 12 aux fréquences de résonance des différents modes propres de la membrane.

L'optimisation de tels actionneurs sont décrits dans le document F. Casset et al, « Piezoelectric membrane actuator design », 12th Int. Conf. On Thermal, Mechanical and Multiphysics Simulation and Experiments in Microelectronics and Microsystems (IEEE Eurosime), 18-20 april2011, pp. 1-5.

En variante, la membrane pourrait être de forme carrée ou rectangulaire.

En variante, un seul actionneur pourrait être mis en oeuvre.

De plus, le ou les actionneurs peuvent être des actionneurs de type électrostatique, magnétique, thermique...

En variante, on pourrait envisager que ce soit le côté du dispositif portant le ou les actionneurs qui présente des propriétés d'adhérence par rapport à un ou plusieurs types de cellules; des moyens pour isoler électriquement les actionneurs du fluide contenant les cellules seraient alors prévus, par exemple une couche isolante électrique.

Chaque type de cellules biologiques peut être caractérisé par une valeur de force d'adhérence par rapport à une surface donnée et des conditions de culture. Les forces d'adhérence des différents types cellulaires sont généralement comprises entre 1 nN et 500 nN.

La température, le pH, la pression d'$O_2$ et la présence de sérum dans le milieu de culture peuvent avoir un impact sur l'adhérence. Par exemple, une température inférieure à 37°C pour des cellules humaines diminue les forces d'adhérence. La présence de sérum dans le milieu de culture favorise quant à elle l'adhérence mais l'aspect quantitatif est très variable d'un type cellulaire à un autre. L'impact sur l'adhérence des conditions de culture dépend du type de surface considéré.

Nous allons donner des exemples de force d'adhérence $F_{adh}$ pour différents types de cellule:

- pour des fibroblastes (NIH/3T3) sur du verre non fonctionnalisé $F_{adh}$ est de l'ordre de 369 nN;
- pour des fibroblastes murins L929 sur un plastique de culture cellulaire, $F_{adh\ est}$ comprise entre 310nN et 390 nN;
- pour des cellules de carcinome cervical humain, qui sont des cellules cancéreuses, sur une surface fonctionnalisée avec de la fibronectine $F_{adh}$ est de l'ordre de 204 nN
- pour des cellules epithéliales normales du col $F_{adh}$ est de l'ordre 100nN,
- pour d'autres types cellulaires, $F_{adh}$ est plus faible, par exemple pour les keratinocytes $F_{adh}$ est de l'ordre de 20 nN.

Le fonctionnement du dispositif de tri va maintenant être décrit.

Une solution contenant en autre les cellules d'intérêt est mise en circulation entre l'entrée 4 et la sortie 6, à la fois les cellules d'intérêt et d'autres cellules contenues dans la solution adhèrent à la surface 12.2 du fait du choix du matériau de celle-ci.

Ensuite, la membrane 12 est mise en vibration à une fréquence donnée qui imprime une accélération aux cellules. Le type de cellules dont l'accélération assure le décollement se décolle et est évacué par le fluide. On peut décider de détacher soit les cellules d'intérêt et de récupérer le fluide sortant de la sortie d'évacuation, soit de détacher les autres cellules. Dans ce dernier cas de figure, en recommençant plusieurs fois la fixation de cellules d'intérêt et le détachement des cellules hors d'intérêt, il est possible d'accumuler les cellules d'intérêt sur la membrane.

La fréquence augmente avec la force d'adhérence. Il sera compris que sont décollées en premier les cellules présentant la force d'adhérence la plus faible par rapport à tous les types de cellules collés sur la surface et que sont décollées les cellules dans l'ordre de leur force d'adhérence croissante.

De manière très avantageuse, la surface du support peut être fonctionnalisée soit en formant une couche de fonctionnalisation par exemple par dépôt, soit en structurant la surface. La fonctionnalisation permet de modifier les forces d'adhérence vis-à-vis de certains types de cellules de manière discriminatoire et ainsi d'optimiser le pouvoir de résolution du dispositif.

Il peut s'agir d'une fonctionnalisation chimique de la surface qui peut se faire par exemple avec des molécules de la matrice extracellulaire comme la fibronectine, le collagène I, mais aussi des composés cationiques comme la Poly-L-Lysine où encore avec des anticorps. Il peut s'agir d'une fonctionnalisation physique, pour cela on modifie la microstructuration de la surface. Une fonctionnalisation physique et une fonctionnalisation chimique peuvent être envisagées.

On peut envisager que les forces d'adhérence entre deux types cellulaires soient inversées par la fonctionnalisation d'une surface, par rapport à la situation sans fonctionnalisation.

Nous allons expliquer comment peut être déterminée la fréquence à laquelle un type de cellules se décolle d'une surface donnée.

En estimant la masse par exemple d'une cellule eucaryote à environ 1 ng, on peut évaluer les ordres de grandeurs de fréquences de vibration de la membrane assurant un décollement des cellules. On considère la cellule biologique comme un système mécanique passif en première approximation.

La fréquence peut s'écrire :

$$f \approx \frac{1}{2\pi} \sqrt{\frac{F_{adh}}{m\delta}} \qquad (I)$$

Avec m la masse d'une cellule d'environ 1 ng,
$F_{adh}$ la force d'adhérence de la cellule pour une surface donnée,
δ l'amplitude de vibration pouvant être modulée entre 0,1 μm et 10 μm.

L'ordre de grandeur des fréquences à appliquer à la membrane est alors compris entre environ 50 Hz et environ10 kHz.

Par ailleurs, la membrane étant destinée à être utilisée en milieu liquide en écoulement, celui-ci applique des forces de cisaillement aux cellules qui ont adhéré à la membrane qui pourraient décoller les cellules et interférer avec le mécanisme de tri par vibration.

Les forces de cisaillement τ peuvent s'écrire de la façon suivante :

$$\tau = \eta \ (\partial v\_\| )/\partial z \sim \eta \ \omega\delta/\delta = \eta\omega$$

Avec $\eta$ la viscosité du milieu, celle-ci est estimée à environ $10^{-3}$m$^2$s$^{-1}$, $v_{//}$ la vitesse parallèle à la surface que l'on estime du même ordre de grandeur que la vitesse de mouvement de la plaque en régime incompressible pour un fluide mouillant et ω la fréquence de vibration de la plaque.

La surface d'une cellule sur laquelle va s'appliquer la force est voisine de 10 μm$^2$.

L'ordre de grandeur des forces de cisaillement appliquées par le fluide est alors compris entre 10 pN et 1 nN, celui-ci est inférieur aux forces d'adhérence des cellules énoncées ci-dessus et pour des cellules cancéreuses, comme par exemple la lignée cancéreuse de carcinome mammaire MCF7 sur du PDMS nanostructuré, la force d'adhérence est voisine de 7 nN. Ainsi il n'y a pas de risque que les cellules soient décrochées par le liquide et non par la mise en vibration de la membrane.

L'estimation précédente repose sur l'hypothèse que la longueur d'onde du premier mode de vibration d'une membrane circulaire est au moins de l'ordre de grandeur de la taille caractéristique de la cellule.

Mais il sera compris que l'invention ne se limite ni au premier mode de vibration, ni à la mise en œuvre d'une membrane circulaire comme cela sera expliqué ci-dessous ni à des ondes dont la longueur d'onde est au moins de l'ordre de grandeur de la taille caractéristique de la cellule. L'invention peut avantageusement mettre en œuvre un ou des modes dont les ondes ont des longueurs d'onde inférieures à la taille de la cellule, par exemple en travaillant à des fréquences de l'ordre du MHz, la longueur d'onde est de l'ordre du μm. Ainsi, ces courtes longueurs d'ondes, vont correspondre à des déformations de la membrane sur des dimensions planaires comparables aux dimensions d'une cellule, voire inférieures. De tels modes permettent très avantageusement de décrocher les points focaux d'adhérence cellulaire. Le décrochement des points focaux d'adhérence présente l'avantage d'augmenter considérablement les chances de survie cellulaire lors du décrochement. En effet les modes de vibration impliquant une force inertielle sur la totalité du corps cellulaire peuvent entrainer des dommages membranaires pour des amplitudes et/ou fréquences élevées.

Dans le cas d'un dispositif comportant une membrane circulaire avec actionnement piézoélectrique, la fréquence de résonance pour les modes de vibration hors plan peut être déterminée par le calcul analytique, comme par exemple l'utilisation de l'équation suivante

$$f_{\mathrm{r}} = \frac{\lambda_n^2 t}{2\pi r^2}\sqrt{\frac{E}{12\rho(1-v^2)}}$$

Avec $\lambda_n$ un paramètre fonction du mode, t l'épaisseur de la membrane, r le rayon de la membrane, E le module d'Young, ρ la densité du matériau de la membrane et v le coefficient de Poisson de la membrane.

Alternativement, la simulation par élément finis peut également permettre de dimensionner le dispositif vibrant afin d'obtenir les fréquences des différents modes. Les logiciels FEM tels que Coventor®, COMSOL® ou ANSYS® peuvent être utilisés.

Par exemple, une membrane peut avoir un rayon compris entre quelques μm, typiquement 5 μm, et quelques centaines de μm, par exemple 5000 μm. L'épaisseur peut être de quelques dizaines de nm à plusieurs μm. A titre d'exemple, une membrane peut avoir un rayon de 500 μm et une épaisseur de 6 μm.

A tire d'exemple uniquement, une membrane ayant un diamètre de 1 cm peut permettre de trier environ 1 million de cellules, par exemple 500000 cellules de chaque type.

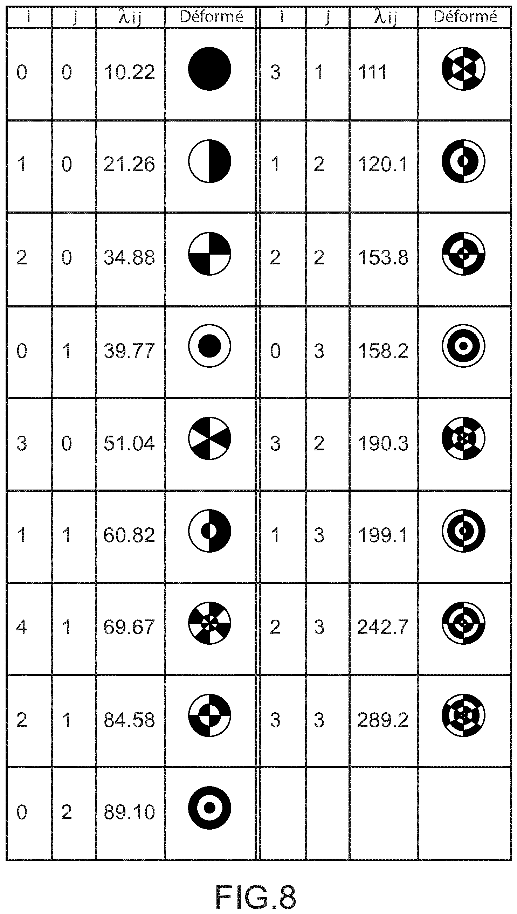

Les membranes circulaires peuvent présenter de nombreux modes, dont les formes sont données par exemple sur la figure 8. i est le nombre de diamètres nodaux et j est le nombre de cercles nodaux sans compter l'encastrement en périphérie de la membrane, λij est un coefficient qui est fonction du mode considéré.

Chacun de ces modes peut être utilisé pour trier des populations de cellules. Il est donc aisé d'adapter les vibrations de la membrane aux types de cellules à trier.

Comme cela a déjà été mentionné ci-dessus, le support vibrant peut avoir toute forme, il peut par exemple être formé par une poutre vibrante encastrée sur un substrat à une des ses extrémités.

Dans un exemple de réalisation représenté sur la figure 3, le support 18 est formé par une plaque de forme rectangulaire. La plaque peut être fixée sur sa tranche sur un à 4 cotés, ou être fixée uniquement par des points de fixation, de type scotch double face ou encoches de fixation, positionnés à des points nodaux. Sa mise en vibration peut également être obtenue par un ou plusieurs actionneurs piézoélectriques 20. La plaque rectangulaire peut être mise en vibration dans son mode de Lamb. Cette configuration permet d'avoir une amplitude de vibration et donc une cartographie de force appliquée aux cellules qui soit homogène sur la surface. Ainsi on assure un décollement homogène des cellules sur toute la surface de la plaque. Par exemple les actionneurs sont positionnés de façon à favoriser le ou les modes recherchés. Le calcul FEM peut être avantageusement utilisé pour optimiser la position et les dimensions des actionneurs.

Sur la figure 3, les actionneurs sont disposés en ligne perpendiculairement à la longueur de la plaque.

De manière générale, le support vibrant peut comporter une ou plusieurs rangées d'actionneurs, chaque rangée comportant au moins un actionneur. Les actionneurs d'une rangée sont séparées les uns des autres d'une distance donnée.

Par exemple, un support vibrant peut comporter une colonne de plusieurs actionneurs séparés par un espace de 500 μm, les actionneurs étant disposés sur un ventre de vibration, ce qui correspond à une zone de maximum de vibration du support

La plaque est dimensionnée de sorte à délivrer la fréquence et le mode de résonance adapté pour détacher la famille de cellule concernée.

A titre d'exemple, nous allons donner un exemple de dimensionnement d'un dispositif permettant de trier deux familles distinctes de cellules sur une plaque en verre mise en vibration par des actionneurs PZT :

- Les fibroblastes (NIH/3T3) présentent des forces d'adhérence de l'ordre de 369nN sur du verre,
- Les keratinocytes présentant des forces d'adhérence de l'ordre de 20nN.

A l'aide de l'équation (I), nous pouvons approximer qu'à partir d'une amplitude de déformation de 1,5μm, un mode de vibration à environ 24,5kHz décollera les fibroblastes et un mode de vibration à environ 5kHz décollera les keratinocytes.

Le support vibrant permettant de trier les fibroblastes et les keratinocytes peut présenter les caractéristiques suivantes: il comporte une plaque en verre ayant une longueur de 82 mm, une largeur de 61 mm et une épaisseur de 700 μm. Il comporte six actionneurs par rangée, chaque actionneur étant de forme rectangulaire dont la longueur s'étend suivant la largeur de la plaque. Les actionneurs ont une longueur de 9 mm et une largeur de 5 mm. La distance séparant deux lignes d'actionneurs est de 4000 μm.

Des analyses modales réalisées à l'aide d'un logiciel de calcul par éléments finis, par exemple le logiciel Coventor® ou Ansys®, permettent également de définir les dimensions suivant les trois directions de l'espace induisant les fréquences désirées.

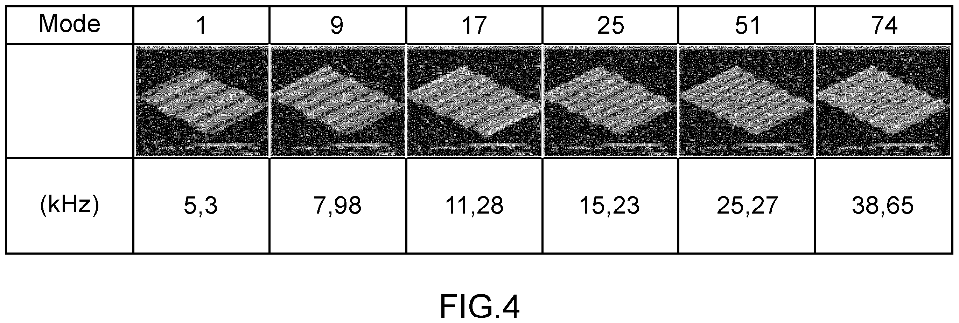

Sur la figure 4 sont représentées plusieurs modes de vibration d'une plaque de verre de 82 mmx61 mm ayant une épaisseur de 700 μm de verre. On constate que le mode 1 à 5,3 kHz permet de décoller les keratinocytes et le mode 51 à 25,27 kHz permet de décoller les fibroblastes.

Ainsi il est possible de déterminer les caractéristiques du support vibrant en un matériau donné pour décoller différents types de famille connaissant leur force d'adhérence à ce matériau donné.

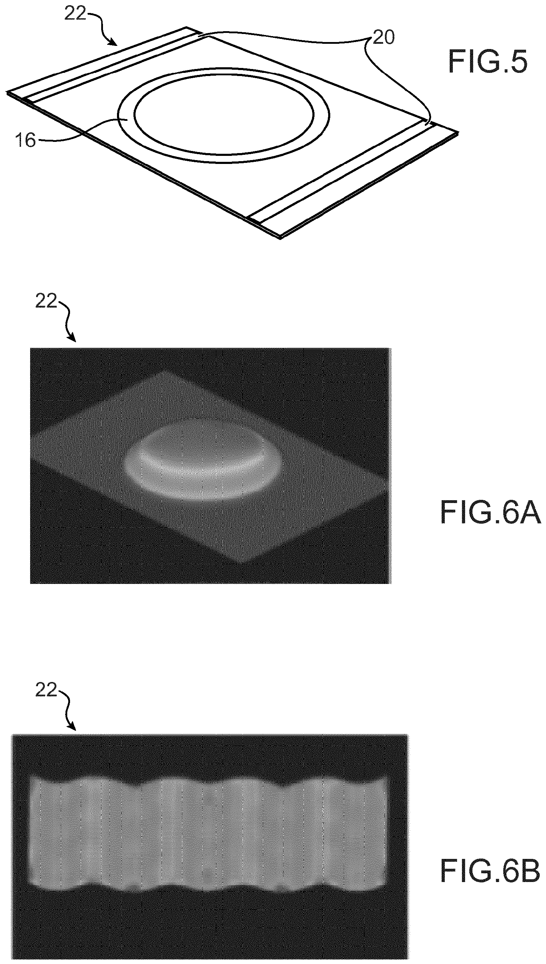

Sur la figure 5, on peut voir un autre exemple de réalisation d'un support vibrant 22 selon l'invention qui associe différents types d'actionneurs pour générer plusieurs modes de vibration. Dans cet exemple, le support est formé par une plaque rectangulaire dans la partie centrale comporte un actionneur similaire 16 à celui d'une membrane circulaire pour générer un premier mode et des actionneurs 20pour générer un mode de Lamb similaire à ceux de la figure 3. Sur la figure 6A, on peut voir le support dans un premier mode de vibration lorsque l'actionneur 16 est activé et sur la figure 6B on peut voir le support dans son mode de Lamb lorsque les actionneurs 20 sont activés.

D'autres combinaisons d'actionneurs sont envisageables.

Chaque dispositif est conçu pour permettre de décrocher un ou plusieurs types de cellules en appliquant une fréquence donnée. Néanmoins il peut être prévu préalablement à l'utilisation du dispositif de tri de calibrer celui-ci afin de déterminer précisément à quelle fréquence chacun des types de cellules de détache du support vibrant. Cette calibration se fait à partir des cellules d'intérêt que l'on souhaite trier avec le dispositif. On désigne par A, B, et C des types de cellules d'intérêt. Le support est réalisé de telle sorte que les cellules adhèrent à la surface de celui-ci.

a) Selon une première étape, on introduit la population A de cellule dans le système. Les cellules adhèrent au support. On effectue ensuite un balayage en fréquence du support vibrant afin de passer en revue les différents modes. On note le mode et la fréquence pour laquelle la population A se détache. La détection des cellules détachées peut se faire par exemple par observation au microscope de la surface considérée, par la récupération du liquide et analyse en microscope des cellules détachées ou en cytométrie en flux avec une comparaison des données avant le tri pour l'évaluation de la pureté du tri.

b) Lors d'une étape suivante, on applique le même protocole que la première étape avec la type de cellules B.

c) On recommence ce protocole avec chaque type de cellule d'intérêt. On connaît désormais pour le dispositif de tri donné les fréquences de détachement de chacun des types de cellules d'intérêt.

d) On vérifie ensuite les fréquences déterminées.

Pour cela, on introduit l'échantillon biologique contenant les types A, B et C à trier. Les cellules adhèrent au support.

On fait ensuite un balayage en fréquence en fonction des modes précédemment déterminés : à chacun de ces modes on récupère les populations de cellule décrochées pour vérifier la pureté du tri. Si chacune des populations récupérées est pure, le dispositif est correctement étalonné, sinon les étapes a) à d) sont répétées. En outre, on peut vérifier si les cellules sont vivantes. Si ce n'est pas le cas on peut modifier la fréquence pour appliquer des ondes de longueur plus courtes et ainsi provoquer le décollement de points focaux et non de toute la cellule d'un coup, comme cela a été décrit ci-dessus. Les actionneurs mis en œuvre sont capables de générer des modes de vibration haute fréquence qui présentent de petites longueurs d'onde.

Le balayage se fait des fréquences les plus basses aux fréquences les plus hautes pour éviter de décrocher toutes les cellules en même temps.

Le dispositif à support vibrant selon l'invention est particulièrement adapté à une mise en œuvre dans un dispositif de tri cellulaire, ce tri pouvant être réalisé de manière entièrement automatique après un étalonnage précis du dispositif.

Néanmoins le dispositif peut avoir d'autres applications, en effet la mise en vibration du support permet de contrôler les propriétés d'adhérence d'une surface par rapport à un ou plusieurs types de cellules. On peut donc envisager de mettre en œuvre l'invention pour empêcher des cellules d'adhérer ou pour décoller un ou plusieurs types de cellules pour en débarrasser la surface du support.

Par exemple, l'invention est mise en œuvre pour réaliser une surface antiadhésive active pour un ou plusieurs types de cellules dans un circuit microfluidique. En régime permanent, on applique une fréquence de vibration à la surface suffisamment élevée pour que tous les types de cellules soient décollés. Par exemple, en connaissant le type de cellules présentant la force d'adhérence la plus élevée par rapport à la surface, on applique une fréquence permettant le décollement de ce type de cellules ou empêchant ce type de cellule d'adhérer à la surface, ce qui provoquera le décollement des autres types de cellules ayant un force d'adhérence inférieure. La surface est ainsi protégée. L'invention présente l'avantage d'offrir une grande capacité d'adaptation. En effet, on peut prévoir par exemple pendant une phase de fonctionnement de protéger une surface en évitant que toute cellule adhère à la surface et pendant une autre phase d'autoriser tous les types de cellules à adhérer à la surface ou certains types de cellules.

En régime commandé, elle permet de détacher des cellules. Par exemple on effectue un contrôle de la surface, par exemple un contrôle optique de la surface, pour détecter des cellules indésirables ayant adhérer à la surface. Si c'est le cas le support est mis en vibration à la fréquence de décollement des cellules indésirables et celles-ci sont décollées. Ce décollement est possible si la fréquence de décollement des cellules indésirables est inférieure à celles des cellules que l'on souhaite conserver.

Un exemple de procédé de réalisation d'un dispositif de tri selon l'invention va maintenant être décrit, celui-ci comportant une membrane circulaire. Il s'agit avantageusement d'un procédé mettant en œuvre des techniques de la microélectronique, ce qui permet de réaliser un dispositif adapté à la taille de cellules. D'autres procédés pourraient être envisagés.

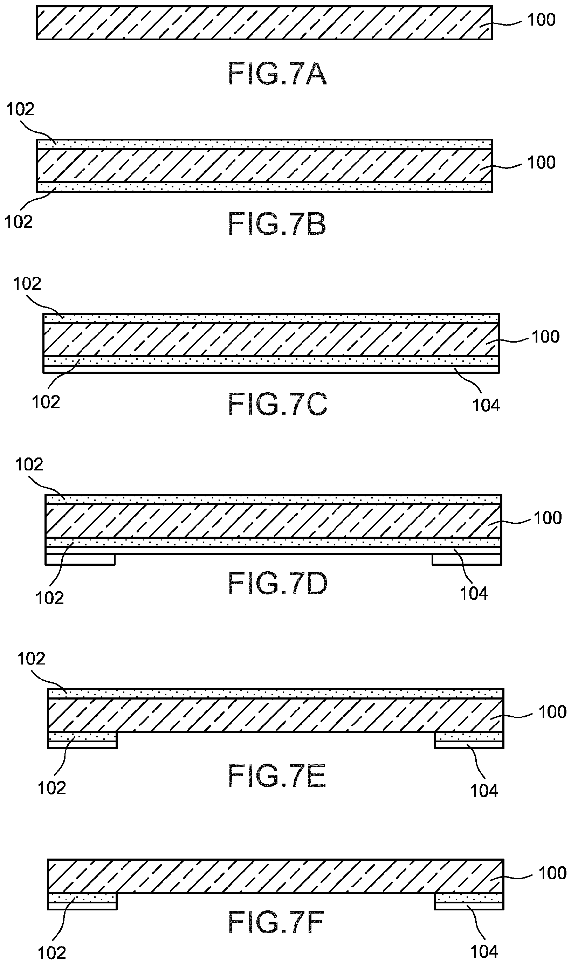

Les étapes sont représentées schématiquement sur les figures 7A à 7S.

Par exemple, on utilise un substrat en silicium 100 représenté sur la figure 7A, ayant par exemple une épaisseur de 725 μm et un diamètre de 200mm de diamètre. Un substrat en verre par exemple peut être envisagé.

Lors d'une première étape, on effectue une oxydation thermique du substrat de sorte à former une couche d'oxyde 102 sur toutes les surfaces du substrat d'une épaisseur de 2 μm par exemple. L'élément ainsi obtenu est représenté sur la figure 7B.

Ensuite, on réalise un masque dur d'oxyde 104 sur la face arrière du substrat. Ce masque a par exemple une épaisseur de 7 μm. Le masque est réalisé en retournant le substrat; en fonction de la composition de dépôt choisie; il est possible de ne déposer le masque que sur cette face. Il peut s'agir par exemple d'un dépôt de type PVD (Physical Vapor Deposition en terminologie anglo-saxonne).L'élément ainsi obtenu est représenté sur la figure 7C.

On effectue ensuite une lithographie sur le masque dur. L'élément ainsi obtenu est représenté sur la figure 7D.

Lors d'une étape suivante, on grave, par exemple par gravure ionique réactive (RIE : Reactive-Ion Etching en terminologie anglo-saxonne), le masque dur et la couche d'oxyde 102 en face arrière de sorte à atteindre la face arrière du substrat 100. L'élément ainsi obtenu est représenté sur la figure 7E.

Lors d'une étape suivante, on retire la couche d'oxyde sur la face avant, par exemple par désoxydation ou gravure chimique. L'élément ainsi obtenu est représenté sur la figure 7F.

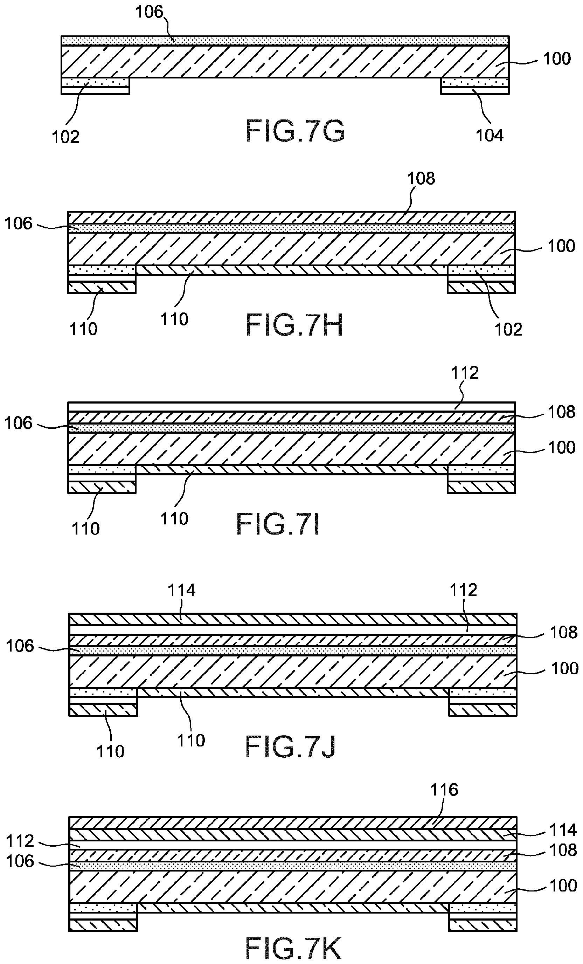

Lors d'une étape suivante, on forme une couche d'oxyde 106 en face avant. Avantageusement, un recuit de densification a lieu par exemple à une température de l'ordre de 800°C.L'élément ainsi obtenu est représenté sur la figure 7G.

Lors d'une étape suivante, on forme une couche 108 en face avant destinée à former la membrane 2, et une couche 110 en face arrière. De préférence, ces couches sont par exemple en polysilicium, en SiC ou en $SiO_2$. L'épaisseur des couches 108, 110 est par exemple comprise entre quelques centaines de nm à plusieurs μm, voire plusieurs dizaines de μm.

Les couches 108, 110sont par exemple réalisées par dépôt chimique en phase vapeur (ou CVD pour Chemical Vapor Déposition en terminologie anglo-saxonne) ou par croissance épitaxiale. De préférence, les contraintes des couches 108, 110 sont maîtrisées.

Les couches 108, 110 peuvent être formées en plusieurs fois. Par exemple, pour une épaisseur de 4 μm, deux couches de 1,5 μm d'épaisseur et 1 couche de 1 μm d'épaisseur sont réalisées successivement.

Avantageusement une étape de recuit a ensuite lieu. L'élément ainsi obtenu est représenté sur la figure 7H.

Lors d'une étape suivante, une couche 112 est formée sur la couche 108, par exemple en SiO$_2$ ou en SiN, ayant par exemple une épaisseur comprise entre quelques centaines de nm et plusieurs μm. La couche 112 est formée par exemple par oxydation thermique ou par dépôt CVD. Avantageusement, un recuit de densification a lieu par exemple à une température de l'ordre de 800°C.

L'élément ainsi obtenu est représenté sur la figure 7I.

Lors d'une étape suivante, on réalise les premier et deuxième actionneurs.

Pour cela on réalise tout d'abord une couche 114 destinée à former les électrodes inférieures des actionneurs, par exemple en Pt ou en Mo. La couche 114 est réalisée par exemple par dépôt sur la couche 112. La couche 114 a par exemple une épaisseur comprise entre quelques dizaines de nm à quelques centaines de nm. L'élément ainsi obtenu est représenté sur la figure 7J.

Une couche de matériau piézoélectrique 116 est ensuite formée sur la couche 114, par exemple en PZT, AIN, ZnO, LNO dont l'épaisseur est par exemple comprise entre quelques centaines de nm à quelques μm.

On réalise ensuite l'électrode supérieure par formation d'une couche 118 sur le matériau piézoélectrique 116, par exemple en Ru ou en Au par exemple d'épaisseur comprise entre quelques dizaines de nm à quelques centaines de nm. L'élément ainsi obtenu est représenté sur la figure 7K.

Ont lieu ensuite des étapes de gravure.

Tout d'abord, la couche 118 est gravée de sorte à délimiter l'actionneur annulaire 8 et l'actionneur en forme de disque 10.

Ensuite, la couche 116 en matériau piézoélectrique est gravée.

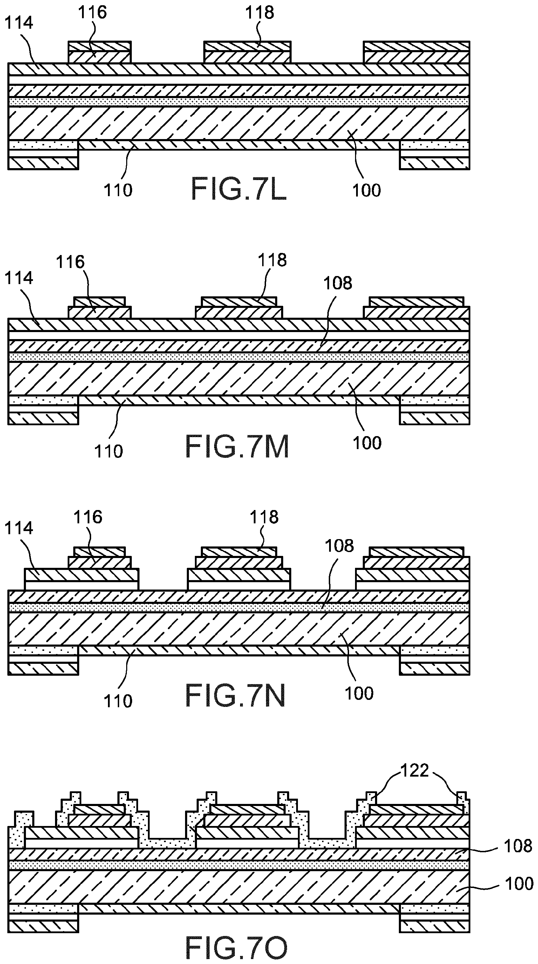

L'élément ainsi obtenu est représenté sur la figure 7L.

Ensuite, on grave à nouveau les portions de couche 118 restantes de sorte à ce qu'elles soient en retrait par rapport aux portions de couche 116.

La couche 114 est ensuite gravée, ainsi que la couche d'oxyde 112. L'élément ainsi obtenu est représenté sur la figure 7M.

De préférence, on réalise un profil en escalier. Celui-ci est obtenu car toutes les couches sont déposées puis gravées, à partir de la couche supérieure, en utilisant des masques de photolithographie différents, le deuxième masque étant plus large que le premier, etc. Cela permet de laisser des marges de sécurité pour éviter le recouvrement de couches, qui pourrait apparaître du fait de l'incertitude de positionnement des masques. On évite ainsi tout court circuit électrique entre les électrodes. L'élément ainsi obtenu est représenté sur la figure 7N.

Lors des étapes suivantes, on réalise des plots de reprise de contact 120. Préalablement on forme une couche 122 de matériau diélectrique, par exemple en SiO$_2$ sur les bords des empilements formés des électrodes inférieure, supérieure et du matériau piézoélectrique, cette couche étant gravée de sorte à dégager partiellement les électrodes inférieure et supérieure. L'élément ainsi obtenu est représenté sur la figure 7O.

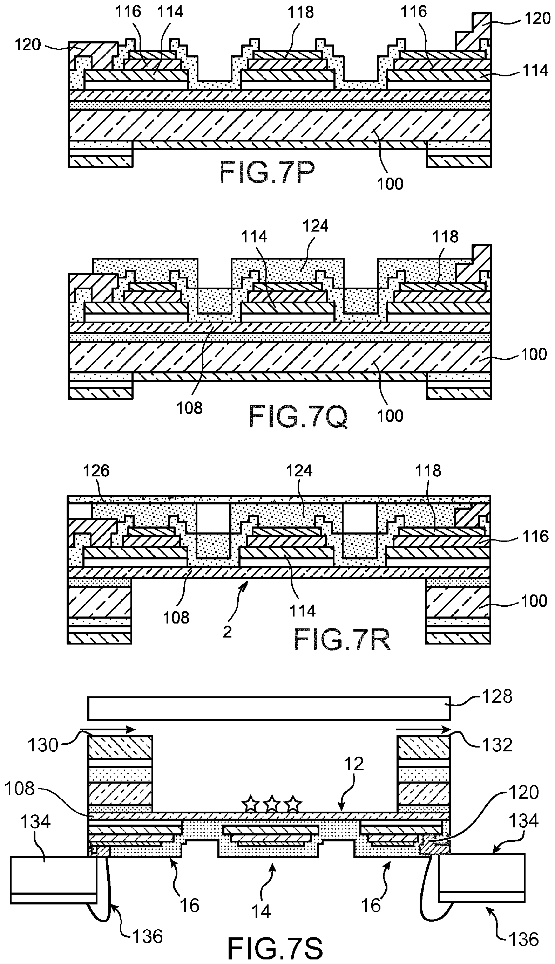

Ensuite, une couche par exemple en AlSi ou en TiAu est formée et est gravée, formant ainsi des plots de contact au niveau des zones où les électrodes ont été dégagées. L'élément ainsi obtenu est représenté sur la figure 7P.

Avantageusement, lors d'une étape suivante on forme une couche de protection 124 sur les actionneurs, par exemple une couche d'oxyde, afin de protéger les actionneurs du contact avec les éléments d'arrêt. L'épaisseur de cette couche peut être comprise entre quelques centaines de nm à quelques μm, par exemple 500nm.

Lors d'une étape suivante la couche 124 est gravée, pour accéder aux reprises de contact.

L'élément ainsi obtenu est représenté sur la figure 7Q.

De préférence, lors d'une étape suivante, on protège les actionneurs, par exemple par le dépôt d'un film sec 126. Ensuite, on grave la face arrière afin de libérer la membrane 2.

On libère la membrane par gravure profonde du substrat par la face arrière jusqu'à atteindre la membrane.

L'élément ainsi obtenu est représenté sur la figure 7R.

Pour pouvoir être utilisé en milieu liquide, un packaging adapté est réalisé. On ferme la cavité formée du côté de la face arrière de la membrane par un capot 128. En effet on utilise préférentiellement la face arrière de la membrane pour effectuer le tri des cellules, ce qui simplifie les connexions électriques des actionneurs situés sur la face avant. Néanmoins les actionneurs étant encapsulés, la face avant pourvue des actionneurs peut également servir pour le tri en adaptant les connexions électriques. En utilisant à la fois la face arrière et la face avant, on double la capacité de tri du dispositif. Les deux faces de la membrane peuvent présenter les mêmes propriétés d'adhérence par rapport aux différents types de cellules ou des propriétés différentes, pour cela on peut fonctionnaliser l'une des faces.

Le capot 128 est par exemple en verre et est par exemple collé sur la couche 110. On prévoit que le capot comporte un orifice d'entrée 130 et un orifice de sortie 132 pour le liquide contenant les cellules

En outre, le dispositif est disposé sur des supports 134 de sorte à suspendre la membrane et à permettre sa mise en vibration. Les supports peuvent être des supports mécaniques ou une partie d'une plaque de circuit électronique. De manière avantageuse, les supports sont également utilisés pour l'alimentation électrique au moyen de microconnexions électriques 136. Les microconnexions électriques sont obtenues par exemple en tendant des microfils, par exemple en or, qui sont soudés sur le plot de contact d'un coté, et sur la carte électronique de l'autre.

L'élément ainsi obtenu est représenté sur la figure 7S. Les cellules sont symbolisées par des étoiles.

Le dispositif peut faire partie d'un microdispositif, par exemple il peut former une partie de la surface d'une chambre microfluidique dans le cas d'un dispositif de tri. Il peut faire partie d'un circuit microfluidique et assurer la protection de l'intérieur de certains des canaux.

Dans l'exemple représenté sur la figure 7S, le support vibrant forme le fond de la chambre mais ceci n'est en aucun cas limitatif il peut former une paroi latérale voir une paroi supérieure. Il sera vérifié alors que ces parois entrent effectivement en contact avec la solution comprenant les cellules.

En outre, on peut envisager qu'un dispositif de tri comporte plusieurs supports vibrants distincts, permettant par exemple de trier un type de cellules par support vibrant.

Grâce à l'invention, on réalise un dispositif présentant des propriétés d'adhérence modifiables pour différents types de cellules permettant de gérer ces différents types de cellules.

## Revendications

1. Dispositif de manipulation d'un ou de plusieurs types de cellules biologiques distinguables à partir de leurs propriétés d'adhérence, comportant :

   - au moins un support (12, 18, 22) comprenant une surface d'accueil permettant l'adhérence desdites cellules, ledit support (12) étant formé par une membrane suspendue, chaque type de cellules biologiques présentant une force d'adhérence pour la surface d'accueil,
   - au moins un actionneur (14, 16, 20) apte à mettre en vibration ladite surface à au moins une fréquence propre de la membrane, et
   - des moyens de commande (CU) dudit actionneur (14, 16, 20) configurés au moins pour mettre en vibration la surface d'accueil à une fréquence propre de la membrane déterminée en fonction de la force d'adhérence d'un type de cellules biologiques provoquant le détachement dudit type de cellules biologiques.

2. Dispositif de manipulation de cellules biologiques selon la revendication 1, les cellules biologiques étant de plusieurs types, dans lequel les moyens de commande (CU) commandent l'actionneur (14, 16, 20) de sorte qu'il mette en vibration ladite surface à plusieurs fréquences données, chacune des fréquences étant choisie de sorte à provoquer le détachement d'au moins un des types de cellules biologiques.

3. Dispositif de manipulation de cellules biologiques selon la revendication 1 ou 2, dans lequel la ou les fréquences est ou sont choisie(s) de sorte que les ondes générées correspondent à des déformations de la surface de l'ordre de la taille des cellules biologiques ou inférieures à la taille des cellules biologiques.

4. Dispositif de manipulation de cellules biologiques selon la revendication 1 à 3, dans lequel ledit actionneur est apte à mettre en vibration la surface à plusieurs fréquences, lesdites différentes fréquences étant appliquées séquentiellement.

5. Dispositif de manipulation de cellules biologiques selon l'une des revendications 1 à 4, dans lequel tout ou partie de la surface est fonctionnalisée de sorte à modifier la force d'adhérence du ou des types de cellules biologiques par rapport à la surface non fonctionnalisée.

6. Dispositif de manipulation de cellules biologiques selon l'une des revendications 1 à 5, dans lequel la plus grande dimension de la membrane est comprise entre quelques $\mu$m et quelques centaines de $\mu$m, de préférence entre 5 $\mu$m et 20000 $\mu$m.

7. Dispositif de manipulation de cellules biologiques selon l'une des revendications 1 à 6, dans lequel les moyens de commande commandent l'actionneur de sorte que le support vibre dans un mode de Lamb.

8. Dispositif de manipulation de cellules biologiques selon l'une des revendications 1 à 7, comportant plusieurs actionneurs (20) répartis sur ou sous la surface du support.

9. Dispositif de manipulation de cellules biologiques selon la revendication 8, dans lequel les moyens de commande (CU) commandent les actionneurs (14, 16, 20) de sorte qu'ils mettent chacun en vibration la surface du support selon un mode différent.

10. Dispositif de manipulation de cellules biologiques selon l'une des revendications 1 à 9, dans lequel l'actionneur ou les actionneurs (14, 16, 20) sont choisis parmi des actionneurs piézoélectriques, ferroélectriques, électrostatiques, magnétiques ou thermiques.

11. Dispositif de manipulation de cellules biologiques selon l'une des revendications 1 à 10, dans lequel l'actionneur est réalisé sur une face du support opposée à la surface destinée à entrer en contact avec les cellules biologiques.

12. Dispositif de manipulation de cellules biologiques selon l'une des revendications 1 à 11 étant un dispositif MEMS et/ou NEMS.

13. Dispositif de tri de cellules biologiques comportant au moins un dispositif de manipulation de cellules biologiques selon l'une des revendications 1 à 12.

14. Dispositif microfluidique comportant au moins un dispositif de manipulation de cellules biologiques selon l'une des revendications 1 à 12 ou un dispositif de tri selon la revendication 13, comportant au moins une entrée d'alimentation (4) en une solution comportant au moins un type de cellules biologiques et au moins une sortie d'évacuation (6), le support étant disposé entre l'entrée d'alimentation (4) et la sortie d'évacuation (6).

15. Procédé de tri de différents types de cellules biologiques contenus dans une solution mettant en œuvre le dispositif de tri selon la revendication 13, comportant les étapes:

   - mise en contact de la solution contenant les différents types de cellules biologiques avec la surface du support,
   - adhérence des cellules de différents types sur la surface du support,
   - mise en vibration de la surface à au moins une fréquence donnée de sorte à détacher au moins l'un des types de cellules,
   - évacuation des cellules détachées.

**Patentansprüche**

1. Vorrichtung zur Manipulation eines oder mehrerer Typen biologischer Zellen, die durch ihre Adhäsionseigenschaften voneinander unterschieden werden können, umfassend:

   - wenigstens einen Träger (12, 18, 22), welcher eine Aufnahmefläche umfasst, die die Adhäsion der Zellen ermöglicht, wobei der Träger (12) durch eine suspendierte Membran gebildet ist, wobei jeder Typ biologischer Zellen eine Adhäsionsstärke für die Aufnahmefläche aufweist,
   - wenigstens einen Aktuator (14, 16, 20), der dazu in der Lage ist, die Oberfläche mit wenigstens einer Eigenfrequenz der Membran in Schwingung zu versetzen, und
   - Steuermittel (CU) des Aktuators (14, 16, 20), die wenigstens dazu konfiguriert sind, die Aufnahmefläche mit einer Eigenfrequenz der Membran in Schwingung zu versetzen, die in Abhängigkeit von der Adhäsionskraft eines Typs biologischer Zellen festgelegt wird, welche die Ablösung des Typs biologischer Zellen bewirkt.

2. Vorrichtung zur Manipulation biologischer Zellen nach Anspruch 1, wobei die biologischen Zellen von einer Vielzahl von Typen sind, wobei Steuermittel (CU) den Aktuator (14, 15, 20) derart steuern, dass er die Oberfläche mit mehreren gegebenen Frequenzen in Schwingung versetzt, wobei jede der Frequenzen derart gewählt ist, dass sie die Ablösung von wenigstens einem der Typen der biologischen Zellen bewirkt.

3. Vorrichtung zur Manipulation biologischer Zellen nach Anspruch 1 oder 2, wobei die Frequenz bzw. die Frequenzen so gewählt ist bzw. sind, dass die erzeugten Wellen Oberflächendeformationen in der Größenordnung der Größe der biologischen Zellen oder weniger als der Größe der biologischen Zellen entsprechen.

4. Vorrichtung zur Manipulation biologischer Zellen nach einem der Ansprüche 1 bis 3, wobei der Aktuator dazu geeignet ist, die Oberfläche mit mehreren Frequenzen in Schwingung zu versetzen, wobei die verschiedenen Frequenzen sequentiell angewandt werden.

5. Vorrichtung zur Manipulation biologischer Zellen nach einem der Ansprüche 1 bis 4, wobei die gesamte oder ein Teil der Oberfläche dazu funktionalisiert ist, die Adhäsionskraft des Typs oder der Typen biologischer Zellen in Bezug auf die nicht funktionalisierte Oberfläche zu modifizieren.

6. Vorrichtung zur Manipulation biologischer Zellen nach einem der Ansprüche 1 bis 5, wobei die größte Abmessung der Membran zwischen einigen $\mu$m und einigen hundert $\mu$m, bevorzugt zwischen 5 $\mu$m und 20000 $\mu$m liegt.

7. Vorrichtung zur Manipulation biologischer Zellen nach einem der Ansprüche 1 bis 6, wobei die Steuerbewegungen den Aktuator derart ansteuern, dass der Träger in einem Lamb-Modus schwingt.

8. Vorrichtung zur Manipulation biologischer Zellen nach einem der Ansprüche 1 bis 7, welche mehrere Aktuatoren (20) umfasst, die auf oder unter der Oberfläche des Trägers verteilt sind.

9. Vorrichtung zur Manipulation biologischer Zellen nach Anspruch 8, wobei die Steuermittel (CU) die Aktuatoren (14, 16, 20) derart steuern, dass sie die Oberfläche des Trägers jeweils in einem anderen Modus in Schwingung versetzen.

10. Vorrichtung zur Manipulation biologischer Zellen nach einem der Ansprüche 1 bis 9, wobei der Aktuator oder die Aktuatoren (14, 16, 20) ausgewählt sind aus piezoelektrischen, ferroelektrischen, elektrostatischen, magnetischen oder thermischen Aktuatoren.

11. Vorrichtung zur Manipulation biologischer Zellen nach einem der Ansprüche 1 bis 10, wobei der Aktuator auf einer Seite des Trägers vorgesehen ist, die der Oberfläche, welche mit den biologischen Zellen in Kontakt kommen soll, gegenüberliegt.

12. Vorrichtung zur Manipulation biologischer Zellen nach einem der Ansprüche 1 bis 11, die eine MEMS- und/oder NEMS-Vorrichtung ist.

13. Sortiervorrichtung für biologische Zellen, welche wenigstens eine Vorrichtung zur Manipulation biologischer Zellen nach einem der Ansprüche 1 bis 12 umfasst.

14. Mikrofluidische Vorrichtung, welche wenigstens eine Vorrichtung zur Manipulation biologischer Zellen nach einem der Ansprüche 1 bis 12 oder einer Sortiervorrichtung nach Anspruch 13 umfasst, welche wenigstens einen Zufuhreinlass (4) für eine Lösung umfasst, die wenigstens einen Typ biologischer Zellen umfasst, und wenigstens einen Auslass (6), wobei der Träger zwischen dem Zufuhreinlass (4) und dem Auslass (6) angeordnet ist.

15. Verfahren zum Sortieren verschiedenerTypen biologischerZellen, die in einer Lösung enthalten sind, unter Verwendung der Sortiervorrichtung nach Anspruch 13, welches die folgenden Schritte umfasst:

- Inkontaktbringen der Lösung, welche die verschiedenen Typen biologischer Zellen enthält, mit der Oberfläche des Trägers,
- Adhäsion der Zellen verschiedener Typen auf der Oberfläche des Trägers,
- Vibrierenlassen der Oberfläche mit wenigstens einer vorgegebenen Frequenz, um wenigstens einen der Zelltypen abzulösen,
- Entfernen der abgelösten Zellen.

**Claims**

1. Device for manipulating one or more types of biological cells which may be distinguished from their adherence properties, comprising:

- at least one support (12, 18, 22) including a reception surface enabling the adherence of said cells, the support (12) being a suspended membrane, each type of biological cells having a adherence force with respect to the reception surface,
- at least one actuator (14, 16, 20) capable of making said surface vibrate at at least one natural frequency of the membrane, and
- control means (CU) of said actuator (14, 16, 20) configured at least to vibrate the reception surface at a natural frequency of the membrane, which is determined as a function of the adherence force of a type of biological cells, causing the detachment of said type of biological cells.

2. Device for manipulating biological cells according to claim 1, the biological cells being of several types, in which the control means (CU) control the actuator (14, 16, 20) such that it makes said surface vibrate at several given frequencies, each of the frequencies being selected so as to cause the detachment of at least one of the types of biological cells.

3. Device for manipulating biological cells according to claim 1 or 2, in which the frequency or the frequencies is or are selected such that the waves generated correspond to deformations of the surface of the order of the size of the biological cells or smaller than the size of the biological cells.

4. Device for manipulating biological cells according to one of the claims 1 to 3, in which said actuator is capable of making the surface vibrate at several frequencies, said different frequencies being applied sequentially.

5. Device for manipulating biological cells according to one of the claims 1 to 4, in which all or part of the surface is functionalised so as to modify the adherence force of the type(s) of biological cells with respect to the non-functionalised surface.

6. Device for manipulating biological cells according to one of the claims 1 to 5, in which the largest dimension of the membrane is comprised between several $\mu$m and several hundreds of $\mu$m, preferably between 5 $\mu$m and 20,000 $\mu$m.

7. Device for manipulating biological cells according to one of the claims 1 to 6, in which the control means control the actuator such that the support vibrates in a Lamb mode.

8. Device for manipulating biological cells according to one of the claims 1 to 7, comprising several actuators (20) distributed on or under the surface of the support.

9. Device for manipulating biological cells according to claim 8, in which the control means control the actuators (14, 16, 20) such that they each make the surface of the support vibrate according to a different mode.

10. Device for manipulating biological cells according to one of the claims 1 to 9, in which the actuator or the actuators (14, 16, 20) are selected from piezoelectric, ferroelectric, electrostatic, magnetic or thermal actuators.

11. Device for manipulating biological cells according to one of the claims 1 to 10, in which the actuator is formed on one face of the support opposite to the surface intended to enter into contact with the biological cells.

12. Device for manipulating biological cells according to one of the claims 1 to 11, being a MEMS and/or NEMS device.

13. Sorting device for sorting biological cells comprising at least one device for manipulating biological cells according to one of the claims 1 to 12.

14. Microfluidic device comprising at least one device for manipulating biological cells according to one of the claims 1 to 12 or a sorting device according to claim 13, comprising at least one supply inlet (4) with a solution comprising at least one type of biological cells and at least one evacuation outlet (6), the support being arranged between the supply inlet (4) and the evacuation outlet (6).

15. Method for sorting different types of biological cell contained in a solution implementing the sorting device according to claim 13, comprising the steps:

    - bringing the solution containing the different types of biological cell into contact with the surface of the support,
    - adherence of cells of different types on the surface of the support,
    - making the surface vibrate at at least one given frequency so as to detach at least one of the types of cells,
    - evacuation of the detached cells.

FIG.1

FIG.2

FIG.3

| Mode | 1 | 9 | 17 | 25 | 51 | 74 |
|---|---|---|---|---|---|---|
| | | | | | | |
| (kHz) | 5,3 | 7,98 | 11,28 | 15,23 | 25,27 | 38,65 |

FIG.4

EP 3 059 300 B1

FIG.5

FIG.6A

FIG.6B

FIG.7A

FIG.7B

FIG.7C

FIG.7D

FIG.7E

FIG.7F

FIG.7G

FIG.7H

FIG.7I

FIG.7J

FIG.7K

114  116  118

FIG.7L

110  100

114  116  118  108

FIG.7M

110  100

114  116  118  108

FIG.7N

110  100

122

108

100

FIG.7O

FIG.7P

FIG.7Q

FIG.7R

FIG.7S

| i | j | $\lambda_{ij}$ | Déformé | i | j | $\lambda_{ij}$ | Déformé |
|---|---|---|---|---|---|---|---|
| 0 | 0 | 10.22 | | 3 | 1 | 111 | |
| 1 | 0 | 21.26 | | 1 | 2 | 120.1 | |
| 2 | 0 | 34.88 | | 2 | 2 | 153.8 | |
| 0 | 1 | 39.77 | | 0 | 3 | 158.2 | |
| 3 | 0 | 51.04 | | 3 | 2 | 190.3 | |
| 1 | 1 | 60.82 | | 1 | 3 | 199.1 | |
| 4 | 1 | 69.67 | | 2 | 3 | 242.7 | |
| 2 | 1 | 84.58 | | 3 | 3 | 289.2 | |
| 0 | 2 | 89.10 | | | | | |

FIG.8

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012140519 A

**Littérature non-brevet citée dans la description**

- **CHANG et al.** Biomimetic technique for adhesion-based collection and separation of cells in a microfluidic channel. *Lab Chip,* 2005, vol. 5, 64-73
- **MANDRUSOV et al.** Membrane-based cell affinity chromatography to retrieve viable cells. *Biotechnol.Prog.,* 1995, vol. 11, 208-213
- **SIN et al.** Enrichment using antibody-coated microfluidic chambers in shear flow: model mixtures of human lymphocytes. *Biotechnology & Bioengineering,* 30 Septembre 2005, vol. 91 (7), 816-826
- **KWON et al.** Label-free, microfluidic separation and enrichment of human breast cancer cells by adhesion difference. *Lab Chip,* 2007, vol. 7, 1461-1468
- **F. CASSET et al.** Piezoelectric membrane actuator design. *12th Int. Conf. On Thermal, Mechanical and Multiphysics Simulation and Experiments in Microelectronics and Microsystems (IEEE Eurosime),* 18 Avril 2011, 1-5